# EUROPÄISCHE PATENTSCHRIFT

(11) **EP 4 074 364 B1**
(45) Veröffentlichungstag und Bekanntmachung des Hinweises auf die Patenterteilung: **11.06.2025**
(21) Anmeldenummer: 22168176.0
(22) Anmeldetag: 13.04.2022
(51) Int. Cl.: A61M 39/10, A61M 39/12, A61M 25/00, A61F 2/94, A61F 2/95

(54) **MEDIZINISCHER TUBUS UND VERBINDUNGSVORRICHTUNG EINER MEDIZINISCHEN VORRICHTUNG**
MEDICAL TUBE AND CONNECTING DEVICE OF MEDICAL DEVICE
TUBE MÉDICAL ET DISPOSITIF DE LIAISON D'UN DISPOSITIF MÉDICAL

(30) Priorität: 16.04.2021 DE 102021109635
(43) Veröffentlichungstag der Anmeldung: 19.10.2022
(73) Patentinhaber: Medi-Globe GmbH, 83101 Rohrdorf-Achenmühle (DE)
(72) Erfinder: EDEN, Ingo, 81479 München (DE)
(74) Vertreter: Hofstetter, Schurack & Partner

(56) Entgegenhaltungen:
- US-A1- 2003 078 467
- US-A1- 2008 264 413
- US-A1- 2011 313 404
- US-A1- 2015 073 525

## Beschreibung

Die Erfindung betrifft einen medizinischen Tubus umfassend mindestens einen ersten und zweiten Teiltubus, wobei die Teiltuben an entsprechenden, zueinander ausgerichteten Tubenenden mittels mindestens einer Verbindungsvorrichtung lösbar miteinander verbindbar sind.

Medizinische Tuben der eingangs genannten Art sind in einer großen Vielfalt bekannt. Es handelt sich dabei beispielsweise um Vorrichtungen zum Zuführen und Positionieren eines Stents, insbesondere zur Zuführung und Positionierung des Stents innerhalb einer menschlichen oder tierischen Körperhöhle. Dabei wird ein üblicherweise schlauchartiges Schubelement - als ein Teiltubus - an dem der Körperöffnung gegenüberliegenden, proximalen Seite des einzuführenden Stents - als weiterer Teiltubus - lösbar befestigt. Über diese Art Verlängerung werden das Einführen und das Platzieren des Stents innerhalb der Körperöffnung oder des Körpergefäßes gesteuert. Nach der Platzierung des Stents an der gewünschten Stelle innerhalb des Körpers muss das Schubelement vom Stent gelöst werden. Dies erfolgt kann manuell, einerseits durch ein ruckartiges Zurückziehen des Schubelements, so dass sich das Schubelement vom Stentende löst. Andererseits sind sogenannte Dekonnektoren bekannt, die koaxial zum Schubelement verlaufen und über dieser geführt sind und an der Verbindungsstelle zwischen dem Stent und dem Schubelement angreifen und bei einer entsprechenden Krafteinwirkung diese voneinander lösen. Aus der EP 2 640 325 B1 ist ein Stentzuführungssystem bekannt bei dem das Schubelement mittels eines Haltefadens an dem Stent lösbar befestigt ist. Die Verbindung ist über eine Schlaufe, die im Schubelement gebildet und durch die ein Führungskatheter geführt wird, gesichert. Zum Lösen der Verbindung zwischen Stent und Schubelement kann durch Zug am Haltefaden ein Knoten des Haltefadens gelöst werden. Auch einfache Verbindungen von Kathetern untereinander oder mit anderen, röhren- oder schlauchartige Elemente umfassende medizinische Vorrichtungen zur Ausbildung eines medizinischen Tubus sind bekannt.

Aus der US 2011/313404 A1, der US 2008/264413 A1 und der US 2003/078467 A1 sind medizinische Tuben, insbesondere Stentzuführungssysteme und Stents, bekannt, bei denen zwei Teiltuben mittels einer Verrastvorrichtung lösbar miteinander verbunden werden können. Die US 2015/073525 A1 beschreibt ein Stenteinführsystem zum Einführen eines Stents, wobei das Stenteinführsystem einen Schiebekatheter, ein Führungselement und ein längliches Stentbefestigungsmaterial aufweist.

Nachteilig an den bekannten medizinischen Tuben ist jedoch, dass hier das Verbinden und Lösen der Teiltuben sehr zeitaufwändig ist. Zudem ist es nicht möglich den Abstand zwischen den Teiltuben optimal einzustellen.

Aufgabe der vorliegenden Erfindung ist es, einen medizinischen Tubus der eingangs genannten Art zu schaffen, welcher eine sichere, schnelle und genaue Positionierung des Tubus oder einer der Teiltuben ermöglicht.

Diese Aufgabe wird durch einen medizinischen Tubus mit den Merkmalen des Patentanspruchs 1 gelöst. Vorteilhafte Ausgestaltungen mit zweckmäßigen Weiterbildungen der Erfindung sind in den Unteransprüchen angegeben.

Die Erfindung betrifft einen medizinischen Tubus umfassend mindestens einen ersten und einen zweiten Teiltubus, wobei die Teiltuben an entsprechenden, zueinander ausgerichteten Tubenenden mittels mindestens einer Verbindungsvorrichtung lösbar miteinander verbindbar sind, wobei die Verbindungsvorrichtung eine Verrastvorrichtung ist. Die Verrastvorrichtung umfasst dabei mindestens ein federnd und/oder elastisch gelagertes Rastelement im Bereich des Tubenendes des ersten Teiltubus und mindestens eine Aufnahmeöffnung im Bereich des Tubenendes in einer Wandung des zweiten Teiltubus oder im Bereich einer an dem Tubenende des zweiten Teiltubus angeordneten Verbindungshülse, derart, dass zur Verbindung der Tubenenden das Rastelement in der Aufnahmeöffnung verrastbar ist und zum Lösen der Verbindung das Rastelement aus der Aufnahmeöffnung bringbar ist. Vorteilhafterweise wird durch die Verbindungsvorrichtung ein medizinischer Tubus bereitgestellt, der eine sichere, schnelle und genaue Positionierung des Tubus selbst oder einer der Teiltuben ermöglicht. Des Weiteren ist eine sichere und einfache Verbindung zwischen den Teiltuben möglich, wobei auch ein unkompliziertes Lösen der Verbindung über die Verrastvorrichtung ermöglicht wird.

Bei dem medizinischen Tubus handelt es sich beispielsweise um eine Vorrichtung zum Zuführen und Positionieren eines Stents in einer Körperhöhle. Der Stent bildet dabei einen ersten Teiltubus, ein so genanntes schlauchartiges Schubelement den weiteren, zweiten Teiltubus des medizinischen Tubus. Die beiden Elemente lassen sich ohne weiteres lösbar miteinander verbinden und erlauben ein einfaches Re-Positionieren des Stents innerhalb der Körperhöhle. Mittels der Verbindungsvorrichtung lässt sich beispielsweise das genannte Schubelement mit dem Stent verbinden, positionieren und positionsgenau in der entsprechenden Körperhöhle ablegen. Im Vergleich zu entsprechenden, bereits bekannten Vorrichtungen zum Zuführen und Positionierung eines Stents müssen hier beispielsweise keine Schlaufen von Haltefäden kompliziert geknotet werden, es gibt zudem kein Spiel zwischen den Teiltuben, nämlich dem Schubelement und dem Stent. Dadurch ist die Bewegungsübertragung von Schubelement auf Stent direkt und präzise. Insbesondere lässt sich der medizinische Tubus bei über die als Verrastvorrichtung ausgebildete Verbindungsvorrichtung insgesamt drehen und schieben.

Dabei ist ein Innendurchmesser des die Aufnahmeöffnung aufweisenden Tubenendes des zweiten Teiltubus größer als ein Außendurchmesser des das Rastelement aufweisenden Tubenendes des ersten Teiltubus. Es ist aber auch möglich, dass ein Innendurchmesser des die Aufnahmeöffnung aufweisenden Tubenendes des zweiten Teiltubus kleiner ist als ein Außendurchmesser des das Rastelement aufweisenden Tubenendes des ersten Teiltubus. Damit ist einerseits gewährleistet, dass die beiden Tubenenden einfach und sicher ineinander geschoben werden, so dass das Rastelement in der entsprechenden Aufnahmeöffnung verrastbar ist. Es besteht aber auch die Möglichkeit, dass ein Innendurchmesser der Verbindungshülse größer ist als ein Außendurchmesser des das Rastelement aufweisenden Tubenendes des ersten Teiltubus. Auch dadurch ist gewährleistet, dass die Verbindungshülse ohne weiteres über das entsprechende Tubenende des zweiten Teiltubus geschoben werden kann, so dass das Rastelement in der entsprechenden Aufnahmeöffnung verrastbar ist.

In weiteren vorteilhaften Ausgestaltungen des erfindungsgemäßen medizinischen Tubus ist das Rastelement kugelförmig, halbkugelförmig, kasten-, würfel- oder vieleckförmig mit oder ohne randliche Abschrägungen ausgebildet. Auch weitere geometrische Formen sind denkbar. Entscheidend ist dabei, dass das Rastelement ohne weiteres in der entsprechenden Aufnahmeöffnung verrastbar ist und auch zum Lösen der Verbindung das Rastelement aus der Aufnahmeöffnung bringbar ist. Das Rastelement kann dabei insbesondere aus bioverträglichen Materialien, wie beispielsweise einem bioverträglichen Kunststoff, Silikon, Metall, Metalllegierung oder eine Kombination daraus bestehen. Auch weitere Materialien sind denkbar.

Erfindungsgemäß ist zudem an einem Außenumfang des Rastelements ein teilweise oder ganz umlaufendes, flexibel ausgebildetes Befestigungselement angeordnet ist, wobei das Befestigungselement zur Befestigung des Rastelementes an oder in einer Wand des ersten Teiltubus ausgebildet ist. Dabei kann das Befestigungselement mit dem Außenumfang des Rastelements form-, kraft- oder stoffschlüssig verbunden sein und/oder das Befestigungselement kann mit der Wand des ersten Teiltubus form-, kraft- oder stoffschlüssig verbunden sein. Des Weiteren besteht die Möglichkeit, dass eine Innenkante des Befestigungselementes in eine am Außenumfang des Rastelements ausgebildete Nut einbringbar ist. Zur Befestigung des Befestigungselementes in einer entsprechenden Ausnehmung der Wand des ersten Teiltubus kann eine Außenkante des Befestigungselementes zumindest derart ausgebildet sein, dass sie zumindest teilweise in eine in der Wand des ersten Teiltubus ausgebildete Nut eingreift. Es besteht aber auch die Möglichkeit, dass ein Randbereich einer Wandöffnung der Wand des ersten Teiltubus zumindest teilweise in eine am Außenumfang des Befestigungselements ausgebildete Nut einbringbar ist. Insgesamt wird deutlich, dass es eine Vielzahl an Möglichkeiten der Befestigung des Befestigungselementes einerseits an dem Rastelement und andererseits an der Wand des ersten Teiltubus beziehungsweise in einer Wandöffnung der Wand des ersten Teiltubus gibt. Beispielsweise kann das Befestigungselement mit dem Außenumfang des Rastelementes verklebt oder verschweißt sein oder eine einfache Steckverbindung ausbilden. Entsprechendes gilt für die Verbindung des Befestigungselementes mit der Wand des ersten Teiltubus.

Das Befestigungselement besteht aus einem elastischen und/oder flexiblen Material. Insbesondere ist das für das Befestigungselement verwendete Material ebenfalls biokompatibel beziehungsweise bioverträglich. Beispielsweise kann das Befestigungselement aus Silikon bestehen oder als flexible Silikonmembran ausgebildet sein. Vorteilhafterweise ermöglicht das Befestigungselement die federnde oder elastische Lagerung des Rastelementes im Bereich des Tubenendes des ersten Teiltubus.

In weiteren vorteilhaften Ausgestaltungen des erfindungsgemäßen medizinischen Tubus wird zur Verbindung der Tubenenden das Rastelement mittels eines innerhalb des medizinischen Tubus führ- und verschiebbaren, schlauch- oder drahtförmigen Elements in der Aufnahmeöffnung gehalten. Bei dem genannten Element kann es sich beispielsweise um einen so genannten Führungsdraht handeln, der innerhalb des Lumen des medizinischen Tubus führ- und verschiebbar ist. Der Durchmesser des Führungsdrahtes beziehungsweise des genannten Elementes ist dabei derart dimensioniert, dass er einerseits ohne weiteres innerhalb des Lumens des medizinischen Tubus geführt werden kann und andererseits das Rastelement sicher in der entsprechenden Aufnahmeöffnung hält. Zum Lösen der Verbindung der Tubenenden wird der Führungskatheter beziehungsweise das genannte Element aus dem Lumen des medizinischen Tubus entfernt, so dass sich die flexibel und/oder elastisch in oder an der Wand des entsprechenden Teiltubus gelagerten Rastelemente aus den entsprechenden Aufnahmeöffnungen leicht entfernen lassen.

In einer weiteren vorteilhaften Ausgestaltung des erfindungsgemäßen medizinischen Tubus ist zur Verriegelung der Verbindung der Tubenenden das Rastelement mittels einer über den Außenumfang des medizinischen Tubus führ- und verschiebbaren, insbesondere rohrförmigen Verriegelungshülse arretierbar. Damit ist eine sichere Verriegelung der Verrastvorrichtung gewährleistet.

In weiteren vorteilhaften Ausgestaltungen des erfindungsgemäßen medizinischen Tubus ist der erste Teiltubus ein medizinischer Stent und der zweite Teiltubus ein Schubelement zur Zuführung und Positionierung des Stents innerhalb einer menschlichen oder tierischen Körperhöhle. Dabei kann die Verriegelung der Verbindung zwischen den beiden Tubenenden über einen schlauchförmigen Führungskatheter erfolgen.

Die vorgenannten Ausgestaltungen des Rastelementes und des damit verbunden Befestigungselementes weisen einen eigenständigen erfinderischen Gehalt auf und können gegebenenfalls auch mit anderen Verbindungsvorrichtungen kombiniert werden.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder von diesen abweichen. Dabei zeigt:
- Fig. 1: eine schematische Darstellung eines erfindungsgemäßen medizinischen Tubus gemäß einer ersten Ausführungsform;
- Fig. 2: eine schematische Darstellung eines Tubenendes eines ersten Teiltubus des medizinischen Tubus gemäß Fig. 1;
- Fig. 3: eine schematische Darstellung eines Rastelementes des medizinischen Tubus gemäß Fig. 1;
- Fig. 4: eine Schnittdarstellung des medizinischen Tubus gemäß Fig. 1 in verbundenem Zustand;
- Fig. 5: eine Darstellung des medizinischen Tubus gemäß Fig. 1 in verbundenem Zustand;
- Fig. 6a: eine schematische Darstellung eines erfindungsgemäßen medizinischen Tubus gemäß einer zweiten Ausführungsform; und
- Fig. 6b: eine weitere schematische Darstellung des medizinischen Tubus gemäß der Fig. 6b.

Im Folgenden werden zwei Ausführungsbeispiele des medizinischen Tubus beschrieben. Dabei handelt es sich um Zuführ- und Positioniersysteme für Stents in tierische oder menschliche Körperhöhlen. Auch andere medizinische Anwendungen des medizinischen Tubus sind denkbar. Des Weiteren besteht die Möglichkeit, den medizinischen Tubus und die beschriebene Verbindungsvorrichtung auch im nichtmedizinischen Bereich, beispielsweise zur Ausgestaltung eines Schlauches umfassend zwei lösbar miteinander zu verbindende Schlauchelemente beziehungsweise Teilschläuche. Auch diese Ausführungsbeispiele sollen als von der Erfindung umfasst gelten.

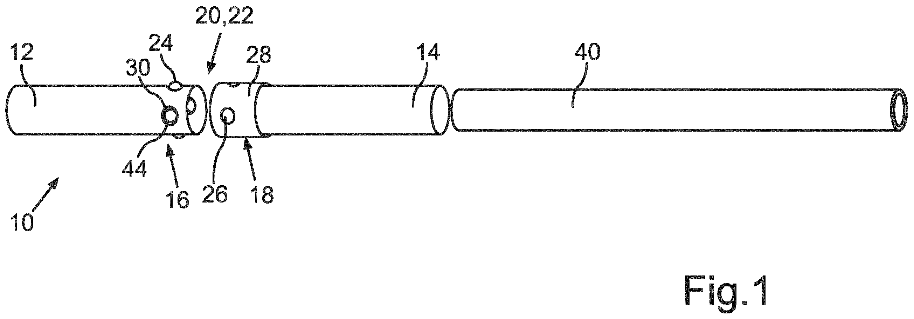

Fig. 1 zeigt eine schematische Darstellung eines medizinischen Tubus 10 gemäß einer ersten Ausführungsform. Der medizinische Tubus 10 umfasst dabei einen ersten und einen zweiten Teiltubus 12, 14 sowie eine Verbindungsvorrichtung 20. Die Verbindungsvorrichtung 20 dient zur lösbaren Verbindung der beiden Teiltuben 12, 14 an ihren zueinander ausgerichteten Tubenenden 16, 18. Man erkennt, dass der medizinische Tubus 10 insgesamt sowie die beiden Teiltuben 12, 14 schlauchartig ausgebildet sind.

Die Verbindungsvorrichtung 20 umfasst eine Verrastvorrichtung 22, wobei die Verrastvorrichtung 22 mehrere federnd beziehungsweise elastisch gelagerte Rastelemente 24 im Bereich des Tubenendes 16 des ersten Teiltubus 12 aufweist. Man erkennt, dass im Bereich einer an dem Tubenende 18 des zweiten Teiltubus 14 angeordneten Verbindungshülse 28 des zweiten Teiltubus 14 entsprechende Aufnahmeöffnungen 26 für die Rastelemente 24 ausgebildet sind. Ein Aufschieben der Verbindungshülse 28 auf das Tubenende 16 des ersten Teiltubus 12 lässt die federnd beziehungsweise elastisch gelagerten Rastelemente 24 in den Aufnahmeöffnungen 26 verrasten. Des Weiteren erkennt man, dass die Rastelemente 24 jeweils über Befestigungselemente 30 in entsprechenden Wandöffnungen 44 des ersten Teiltubus 12 befestigt sind. Zur Verriegelung der Verbindung zwischen dem ersten und dem zweiten Teiltubus 12, 14 wird nach einem Einrasten der Rastelemente 24 in den entsprechenden Aufnahmeöffnungen 26 ein schlauchartiges Element 40, insbesondere ein Führungsdraht, in das Lumen des medizinischen Tubus 10 eingeführt. Der Außenumfang des Elementes 40 drückt dann die Rastelemente 24 in Richtung des Außenumfangs der Verbindungshülse 28 und fixiert die Rastelemente 24 in den Aufnahmeöffnungen 26. Zum Lösen der Verbindung wird das Element 40 aus dem Lumen des medizinischen Tubus 10 entfernt, so dass die elastisch beziehungsweise federnd gelagerten Rastelemente 24 wieder in Richtung des Innenumfangs des ersten Teiltubus 12 und damit aus den Aufnahmeöffnungen 26 bewegt werden können. Alle Elemente des medizinischen Tubus 10 bestehen aus biokompatiblem beziehungsweise bioverträglichem Material. Insbesondere kommen hier entsprechende Kunststoffe, Silikone und Metalle oder Metalllegierungen zur Anwendung.

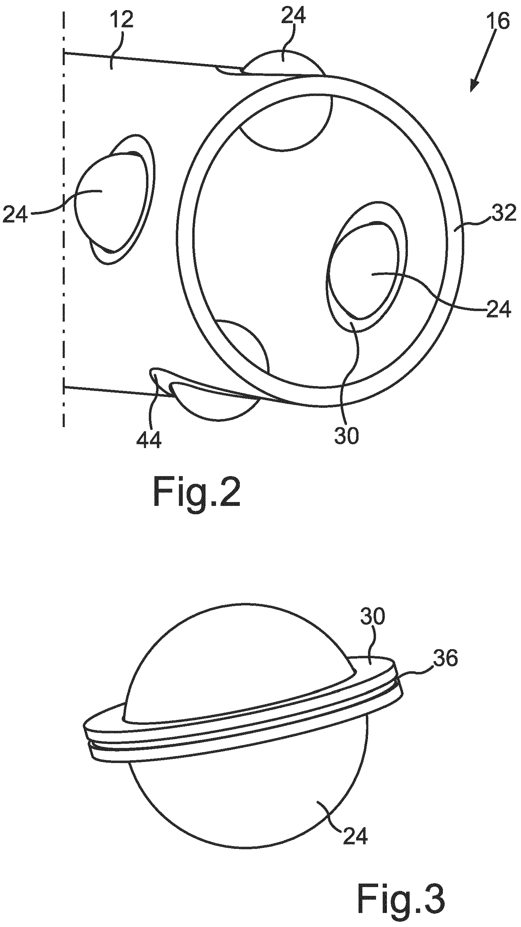

Fig. 2 zeigt eine schematische Darstellung des Tubenendes 16 des ersten Teiltubus 12. Man erkennt, dass die Rastelemente 24 kugelförmig ausgebildet sind und in einer Wandöffnung 44 des ersten Teiltubus 12 angeordnet sind. Hierzu ist ein Befestigungselement 30, welches sich in dem dargestellten Ausführungsbeispiel ringförmig um den Außenumfang des kugelförmigen Rastelementes 24 legt. Die Verbindung zwischen dem ring- und scheibenförmigen Befestigungselement 30 und dem kugelförmigen Rastelement 24 kann dabei form-, kraft- oder stoffschlüssig ausgebildet sein. Zumindest ist das Befestigungselement 30 elastisch beziehungsweise flexibel ausgebildet, so dass eine Relativbewegung des Rastelementes 24 gegenüber der Wand 32 des ersten Teiltubus 12 möglich ist. In dem dargestellten Ausführungsbeispiel besteht das ringförmige Befestigungselement 30 aus Silikon.

Fig. 3 zeigt eine schematische Darstellung des Rastelementes 24 des medizinischen Tubus 10. Man erkennt, dass in der Außenkante des ringförmigen Befestigungselementes 30 eine Nut 36 ausgebildet ist. In diese Nut 36 ist eine Innenkante der Wandöffnung 44 der Wand 32 des ersten Teiltubus 12 einbringbar. Andere Befestigungsmöglichkeiten des Befestigungselementes in oder an der Wand 32 sind jedoch denkbar. Insbesondere können diese form-, kraft- oder stoffschlüssig ausgebildet sein.

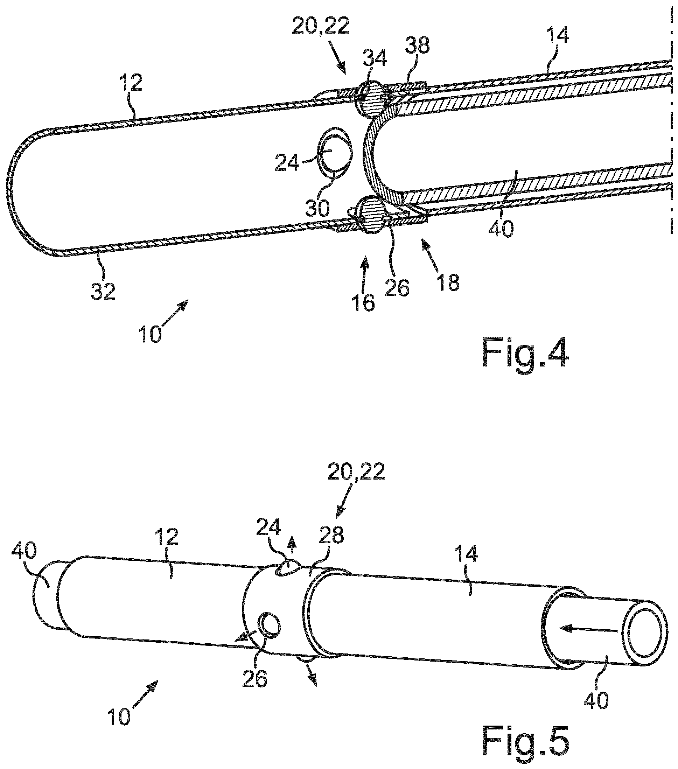

Fig. 4 zeigt eine Schnittdarstellung des medizinischen Tubus 10 gemäß Fig. 1 mit miteinander verbundenen Teiltuben 12, 14. Man erkennt, dass die kugelförmigen Rastelemente mit den Aufnahmeöffnungen 26 der Verbindungshülse 28 verrasten. Zur Fixierung dieser Verraststellung drückt das schlauchförmige Element 40, welches innerhalb des Lumens des medizinischen Tubus 10 geführt ist, gegen die Rastelemente 24 und verankern diese in den entsprechenden Aufnahmeöffnungen 26 der Verbindungshülse 28.

Des Weiteren erkennt man, dass das ringförmige Befestigungselement 30 in einer am Außenumfang des Rastelementes 24 ausgebildeten Nut 34 eingebracht und befestigt ist.

Fig. 5 zeigt in einer schematischen Darstellung den medizinischen Tubus 10 mit miteinander verbundenen Teiltuben 12, 14. Es wird deutlich, dass das schlauchförmige Element 40, insbesondere ein Führungskatheter, der innerhalb des Lumens des medizinischen Tubus 10 geführt ist, die kugelförmigen Rastelemente 24 in die Aufnahmeöffnungen 26 drückt und diese in dieser Stellung verriegelt.

Das in den Figuren 1 bis 5 dargestellte Ausführungsbeispiel des medizinischen Tubus 10 kann auch als Stent-Positionierungs-System bezeichnet werden. Der erste Teiltubus 12 ist dabei als Stent ausgebildet und weist die elastisch gelagerten Rastelemente 24 auf. Der zweite Teiltubus ist als so genannter "Pusher" ausgebildet, der zur Positionierung des Stents innerhalb einer tierischen oder menschlichen Körperhöhle dient. Ein Ende des schlauchförmigen Pushers weist dabei die Verbindungshülse 28 mit den Aufnahmeöffnungen 26 auf. Zur Verbindung des Pushers mit dem Stent wird die Verbindungshülse 28 über das Tubenende 16 des Stents sowie die kugelförmigen Rastelemente 24 geschoben. Durch die flexible beziehungsweise elastische Lagerung der Rastelemente 24 können diese den Weg in Vorschubrichtung der Verbindungshülse 28 freigeben. Beim Überfahren der Rastelemente 24 durch die Verbindungshülse 28 werden diese in das Innenlumen des Stents gedrückt und geben so den Weg für die Verbindungshülse 28 frei. Durch die in der Verbindungshülse ausgebildeten Aufnahmeöffnungen 26 können die Rastelemente 24 wieder zurück in ihre Ausgangsposition gleiten. Wird nun das ebenfalls schlauchförmige Element 40, nämlich ein Führungskatheter, in den Stent beziehungsweise ersten Teiltubus 12 eingeführt, werden die kugelförmigen Rastelemente 24 wiederum nach außen gedrückt und füllen die Aufnahmeöffnungen 26 in der Verbindungshülse 28 des Pushers beziehungsweise zweiten Teiltubus 14 aus. Damit wird der Weg zurück in die Ausgangsposition durch den Führungskatheter gesperrt, das System ist verriegelt. Nach einer entsprechenden Verriegelung des Systems kann der Stent innerhalb der Körperhöhle positioniert werden. Wenn die richtige Position innerhalb der Körperhöhle erreicht ist, wird der Führungskatheter beziehungsweise das schlauchförmige Element 40 entfernt und die Verriegelung so geöffnet. Der Pusher beziehungsweise der zweite Teiltubus 14 mit der Verbindungshülse 28 kann von dem Tubenende 16 des ersten Teiltubus 12 entfernt werden. Der Stent beziehungsweise erste Teiltubus 12 ist abgelegt.

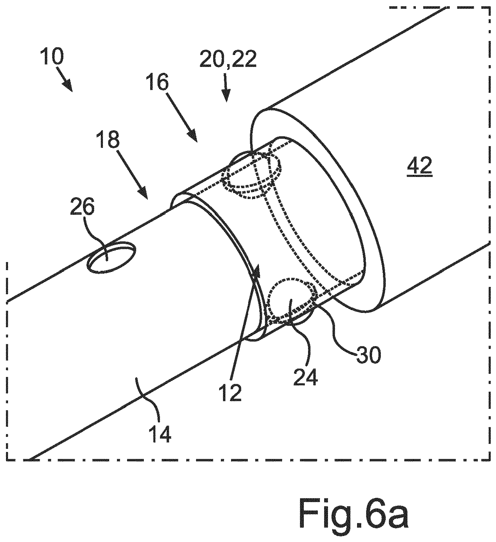

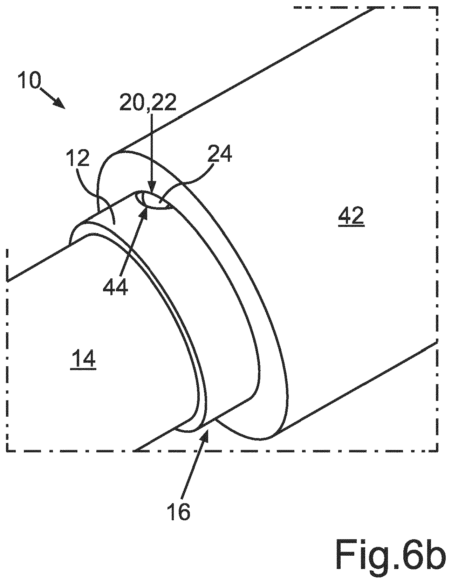

Die Figuren 6a und 6b zeigen schematische Darstellungen eines medizinischen Tubus 10 gemäß einer zweiten Ausführungsform. Man erkennt, dass der erste Teiltubus 12 mit den Rastelementen 24 derart ausgebildet ist, dass ein zweiter Teiltubus 14, der in seiner Wandung entsprechende Aufnahmeöffnungen 26 für die kugelförmigen Rastelemente 24 aufweist, in das Lumen des schlauchförmigen ersten Teiltubus 12 eingeführt werden kann. Da die kugelförmigen Rastelemente 24 auch hier wiederum mittels entsprechender ringförmiger Befestigungselemente 30 flexibel beziehungsweise elastisch an dem Tubenende 16 angeordnet sind, kann durch einen Vorschub des Tubenendes 18 des zweiten Teiltubus 14 in Richtung des Tubenendes 16 des ersten Teiltubus 12 die Rastelemente 24 zunächst in Richtung der Außenseite des ersten Teiltubus 12 durch das Tubenende 18 des zweiten Teiltubus 14 bewegt werden. Sobald die Rastelemente 24 mit den Aufnahmeöffnungen 26 in Deckung sind, fallen diese wieder zurück in ihre Ausgangsposition und füllen so die Aufnahmeöffnungen 26 aus. Zum Verriegeln der Verbindungsvorrichtung 20 beziehungsweise der Verrastvorrichtung 22 wird dann anschließend eine Verriegelungshülse 42 über den Außenumfang des Tubenendes 16 des ersten Teiltubus 12 geschoben (vgl. Fig. 6b). Dadurch werden die Rastelemente 24 in den Aufnahmeöffnungen 26 verriegelt. Aus Fig. 6b ist zudem erkennbar, dass die Rastelemente 24 beziehungsweise die Befestigungselemente 30 wiederum in einer Wandöffnung 44 des ersten Teiltubus angeordnet und befestigt sind.

Durch ein Entfernen der Verriegelungshülse 42 federn die Rastelemente 24 wieder in Richtung des Außenumfangs des ersten Teiltubus 12, so dass sie aus ihrer Verrastung mit den Aufnahmeöffnungen 26 des zweiten Teiltubus 14 gebracht werden können. Der erste Teiltubus 12 und/oder die Verriegelungshülse 42 kann beispielsweise in einen Griff einer medizinischen Vorrichtung integriert sein.

### Bezugszeichenliste:

- 10: Medizinischer Tubus
- 12: Erster Teiltubus
- 14: Zweiter Teiltubus
- 16: Tubenende
- 18: Tubenende
- 20: Verbindungsvorrichtung
- 22: Verrastvorrichtung
- 24: Rastelement
- 26: Aufnahmeöffnung
- 28: Verbindungshülse
- 30: Befestigungselement
- 32: Wand
- 34: Nut
- 36: Nut
- 38: Randbereich
- 40: Element
- 42: Verriegelungshülse
- 44: Wandöffnung

## Patentansprüche

1. Medizinischer Tubus (10) umfassend mindestens einen ersten und zweiten Teiltubus (12, 14), wobei die Teiltuben (12, 14) an entsprechenden, zueinander ausgerichteten Tubenenden (16, 18) mittels mindestens einer Verbindungsvorrichtung (20) lösbar miteinander verbindbar sind, wobei die Verbindungsvorrichtung (20) eine Verrastvorrichtung (22) ist und die Verrastvorrichtung (22) mindestens ein federnd oder elastisch gelagertes Rastelement (24) im Bereich des Tubenendes (16) des ersten Teiltubus (12) und mindestens eine Aufnahmeöffnung (26) in einer Wandung des zweiten Teiltubus (14) im Bereich des Tubenendes (18) des zweiten Teiltubus (14) oder im Bereich einer an dem Tubenende (18) des zweiten Teiltubus (14) angeordneten Verbindungshülse (28) umfasst, derart, dass zur Verbindung der Tubenenden (16, 18) das Rastelement (24) in der Aufnahmeöffnung (26) verrastbar ist und zum Lösen der Verbindung das Rastelement (24) aus der Aufnahmeöffnung (26) bringbar ist, wobei
ein Innendurchmesser des die Aufnahmeöffnung (26) aufweisenden Tubenendes (18) des zweiten Teiltubus (14) größer ist als ein Außendurchmesser des das Rastelement (24) aufweisenden Tubenendes (16) des ersten Teiltubus (12) oder dass ein Innendurchmesser des die Aufnahmeöffnung (26) aufweisenden Tubenendes (18) des zweiten Teiltubus (14) kleiner ist als ein Außendurchmesser des das Rastelement (24) aufweisenden Tubenendes (16) des ersten Teiltubus (12) oder dass ein Innendurchmesser der Verbindungshülse (28) größer ist als ein Außendurchmesser des das Rastelement (24) aufweisenden Tubenendes (16) des ersten Teiltubus (12), **dadurch gekennzeichnet,**
**dass** an einem Außenumfang des Rastelements (24) ein teilweise oder ganz umlaufendes, flexibel ausgebildetes Befestigungselement (30) angeordnet ist, wobei das Befestigungselement (30) zur Befestigung des Rastelements (24) an oder in einer Wand (32) des ersten Teiltubus (12) ausgebildet ist, wobei das Befestigungselement (30) aus einem elastischen und/oder flexiblen Material besteht.

2. Medizinischer Tubus (10) nach Anspruch 1, **dadurch gekennzeichnet, dass** das Rastelement (24) kugelförmig, halbkugelförmig, kasten-, würfel- oder vieleckförmig mit oder ohne randliche Abschrägungen ausgebildet ist.

3. Medizinischer Tubus (10) nach Anspruch 1, **dadurch gekennzeichnet, dass** das Befestigungselement (30) mit dem Außenumfang des Rastelements (24) form-, kraft- oder stoffschlüssig verbunden ist und/oder dass das Befestigungselement (30) mit der Wand (32) des ersten Teiltubus (12) form-, kraft- oder stoffschlüssig verbunden ist.

4. Medizinischer Tubus (10) nach Anspruch 3, **dadurch gekennzeichnet, dass** eine Innenkante des Befestigungselements (30) in eine am Außenumfang des Rastelements (24) ausgebildete Nut (34) einbringbar ist.

5. Medizinischer Tubus (10) nach Anspruch 3 oder 4, **dadurch gekennzeichnet, dass** eine Außenkante des Befestigungselements (30) zumindest teilweise in eine in der Wand (32) des ersten Teiltubus (12) ausgebildete Nut oder ein Randbereich (38) einer Wandöffnung (44) der Wand (32) des ersten Teiltubus (12) zumindest teilweise in eine am Außenumfang des Befestigungselements (30) ausgebildete Nut (36) einbringbar ist.

6. Medizinischer Tubus (10) nach einem der Ansprüche 1 oder 3 bis 5, **dadurch gekennzeichnet, dass** das Befestigungselement (30) aus Silikon besteht oder als flexible Silikonmembran ausgebildet ist.

7. Medizinischer Tubus (10) nach einem der vorhergehenden Ansprüche, **dadurch gekennzeichnet, dass** zur Verriegelung der Verbindung der Tubenenden (16, 18) das Rastelement (24) mittels eines innerhalb eines Lumens des medizinischen Tubus (10) führ- und verschiebbaren, schlauch- oder drahtförmigen Elements (40) in der Aufnahmeöffnung (26) gehalten wird.

8. Medizinischer Tubus (10) nach einem der vorhergehenden Ansprüche, **dadurch gekennzeichnet, dass** zur Verriegelung der Verbindung der Tubenenden (16, 18) das Rastelement (24) mittels einer über den Außenumfang des medizinischen Tubus (10) führ- und verschiebbaren, insbesondere rohrförmigen, Verriegelungshülse (42) in der Aufnahmeöffnung (26) gehalten wird.

## Claims

1. A medical tube (10) including at least one first and second partial tube (12, 14), wherein the partial tubes (12, 14) are detachably connectable to each other at corresponding tube ends (16, 18) aligned with each other by means of at least one connecting device (20), wherein the connecting device (20) is a latching device (22) and the latching device (22) includes at least one resiliently or elastically supported latching element (24) in the area of the tube end (16) of the first partial tube (12) and at least one receiving opening (26) in the area of the tube end (18) of the second partial tube (14) or in the area of a connecting sleeve (28) arranged at the tube end (18) of the second partial tube (14), such that the latching element (24) can be latched in the receiving opening (26) for connecting the tube ends (16, 18) and the latching element (24) can be brought out of the receiving opening (26) for detaching the connection, wherein an internal diameter of the tube end (18) of the second partial tube (14) comprising the receiving opening (26) is larger than an external diameter of the tube end (16) of the first partial tube (12) comprising the latching element (24) or that an internal diameter of the tube end (18) of the second partial tube (14) comprising the receiving opening (26) is smaller than an external diameter of the tube end (16) of the first partial tube (12) comprising the latching element (24) or wherein an internal diameter of the connecting sleeve (28) is larger than an external diameter of the tube end (16) of the first partial tube (12) comprising the latching element (24), **characterized in that** a partially or completely circumferential, flexibly formed attaching element (30) is arranged on an outer circumference of the latching element (24), wherein the attaching element (30) is formed for attaching the latching element (24) on or in a wall (32) of the first partial tube (12), wherein the attaching element (30) consists of a resilient and/or flexible material..

2. The medical tube (10) according to claim 1, **characterized in that** the latching element (24) is spherically, hemispherically, box-shaped, cubically or polygonally formed with or without chamfers at the edge.

3. The medical tube (10) according to claim 1, **characterized in that** the attaching element (30) is connected to the outer circumference of the latching element (24) in form-fit, force-fit or firmly bonded manner and/or that the attaching element (30) is connected to the wall (32) of the first partial tube (12) in form-fit, force-fit or firmly bonded manner.

4. The medical tube (10) according to claim 3, **characterized in that** an inner edge of the attaching element (30) can be introduced into a groove (34) formed on the outer circumference of the latching element (24).

5. The medical tube (10) according to claim 3 or 4, **characterized in that** an outer edge of the attaching element (30) can be at least partially introduced into a groove formed in the wall (32) of the first partial tube (12) or an edge area (38) of a wall opening (44) of the wall (32) of the first partial tube (12) can be at least partially introduced into a groove (36) formed on the outer circumference of the attaching element (30).

6. The medical tube (10) according to any one of the preceding claims 1 or 3 to 5, **characterized in that** the attaching element (30) is composed of silicone or is formed as a flexible silicone membrane.

7. The medical tube (10) according to any one of the preceding claims,
**characterized in that** the latching element (24) is retained in the receiving opening (26) by means of a hose- or wire-shaped element (40) guidable and displaceable within a lumen of the medical tube (10) for locking the connection of the tube ends (16, 18).

8. The medical tube (10) according to any one of the preceding claims,
**characterized in that** the latching element (24) is retained in the receiving opening (26) by means of an, in particular tubular, locking sleeve (42) guidable and displaceable over the outer circumference of the medical tube (10) for locking the connection of the tube ends (16, 18).

## Revendications

1. Tube médical (10) comprenant au moins un premier et un second tube partiel (12, 14), les tubes partiels (12, 14) pouvant être assemblés l'un à l'autre de façon détachable à des extrémités de tube (16, 18) correspondantes, alignées l'une par rapport à l'autre, au moyen d'au moins un dispositif d'assemblage (20), le dispositif d'assemblage (20) étant un dispositif d'enclenchement (22) et le dispositif d'enclenchement (22) comprenant au moins un élément d'enclenchement (24) monté de manière à faire ressort ou élastiquement dans la zone de l'extrémité de tube (16) du premier tube partiel (12) et au moins une ouverture de réception (26) dans une paroi du second tube partiel (14) dans la zone de l'extrémité de tube (18) du second tube partiel (14) ou dans la zone d'une douille d'assemblage (28) agencée sur l'extrémité de tube (18) du second tube partiel (14), de telle sorte que l'élément d'enclenchement (24) peut être enclenché dans l'ouverture de réception (26) pour assembler les extrémités de tube (16, 18) et peut être sorti de l'ouverture de réception (26) pour libérer l'assemblage de l'élément d'enclenchement (24),
un diamètre intérieur de l'extrémité de tube (18) du second tube partiel (14) comportant l'ouverture de réception (26) est supérieur à un diamètre extérieur de l'extrémité de tube (16) du premier tube partiel (12) comportant l'élément d'enclenchement (24), ou un diamètre intérieur de l'extrémité de tube (18) du second tube partiel (14) comportant l'ouverture de réception (26) est inférieur à un diamètre extérieur de l'extrémité de tube (16) du premier tube partiel (12) comportant l'élément d'enclenchement (24), ou un diamètre intérieur de la douille d'assemblage (28) est supérieur à un diamètre extérieur de l'extrémité de tube (16) du premier tube partiel (12) comportant l'élément d'enclenchement (24), **caractérisé en ce qu'**
un élément de fixation (30) réalisé de manière souple, partiellement ou complètement circonférentiel, est agencé sur une circonférence extérieure de l'élément d'enclenchement (24), l'élément de fixation (30) étant conçu pour fixer l'élément d'enclenchement (24) sur ou dans une paroi (32) du premier tube partiel (12), l'élément de fixation (30) étant constitué d'un matériau élastique et/ou souple.

2. Tube médical (10) selon la revendication 1, **caractérisé en ce que** l'élément d'enclenchement (24) est réalisé en forme sphérique, hémisphérique, en forme de bloc, cubique ou polygonale avec ou sans biseaux au niveau du bord.

3. Tube médical (10) selon la revendication 1, **caractérisé en ce que** l'élément de fixation (30) est assemblé à la circonférence extérieure de l'élément d'enclenchement (24) par complémentarité de formes, de manière forcée ou par liaison de matière et/ou **en ce que** l'élément de fixation (30) est assemblé à la paroi (32) du premier tube partiel (12) par complémentarité de formes, de manière forcée ou par liaison de matière.

4. Tube médical (10) selon la revendication 3, **caractérisé en ce qu'**un bord intérieur de l'élément de fixation (30) peut être introduit dans une rainure (34) formée sur la circonférence extérieure de l'élément d'enclenchement (24).

5. Tube médical (10) selon la revendication 3 ou 4, **caractérisé en ce qu'**un bord extérieur de l'élément de fixation (30) peut être introduit au moins partiellement dans une rainure formée dans la paroi (32) du premier tube partiel (4) ou une zone de bordure (38) d'une ouverture de paroi (30) de la paroi (32) du premier tube partiel (38) peut être introduite au moins partiellement dans une rainure (36) formée sur la circonférence extérieure de l'élément de fixation (30).

6. Tube médical (10) selon l'une des revendications 1 ou 3 à 5, **caractérisé en ce que** l'élément de fixation (30) est en silicone ou est réalisé sous la forme d'une membrane souple en silicone.

7. Tube médical (10) selon l'une des revendications précédentes, **caractérisé en ce que** l'élément d'enclenchement (24) est maintenu dans l'ouverture de réception (26) au moyen d'un élément (40) en forme de tube ou de fil, pouvant être guidé et déplacé dans une lumière du tube médical (10), afin de bloquer l'assemblage des extrémités de tube (16, 18).

8. Tube médical (10) selon l'une des revendications précédentes, **caractérisé en ce que** l'élément d'enclenchement (24) est maintenu dans l'ouverture de réception (26) au moyen d'une douille de verrouillage (42), en particulier de forme tubulaire, pouvant être guidée et déplacée sur la circonférence extérieure du tube médical (10), afin de bloquer l'assemblage des extrémités de tube (16, 18).
